# EUROPÄISCHE PATENTSCHRIFT

(11) **EP 1 828 102 B1**
(45) Veröffentlichungstag und Bekanntmachung des Hinweises auf die Patenterteilung: **07.05.2014**
(21) Anmeldenummer: 05815808.0
(22) Anmeldetag: 03.12.2005
(51) Int. Cl.: C07C 231/02, C07C 233/05

(54) **VERFAHREN ZUR HERSTELLUNG VON N,N-DIMETHYLACETAMID (DMAC)**
METHOD FOR THE PRODUCTION OF N,N-DIMETHYLACETAMIDE (DMAC)
PROCEDE POUR PRODUIRE DU N,N-DIMETHYLACETAMIDE (DMAC)

(30) Priorität: 06.12.2004 DE 102004058886
(43) Veröffentlichungstag der Anmeldung: 05.09.2007
(73) Patentinhaber: BASF SE, 67056 Ludwigshafen (DE)
(72) Erfinder: GRAFMANS, Horst, 67098 Bad Dürkheim (DE); MAAS, Steffen, 55270 Bubenheim (DE); WECK, Alexander, 67251 Freinsheim (DE); RÜTTER, Heinz, 2950 Kapellen (BE); SCHULZ, Michael, 67550 Worms (DE); ROSS, Karl-Heinz, 67269 Grünstadt (DE)
(86) Internationale Anmeldenummer: PCT/EP2005/012982
(87) Internationale Veröffentlichungsnummer: WO 2006/061159

(56) Entgegenhaltungen:
- US-A- 4 258 200

## Beschreibung

Die vorliegende Erfindung betrifft ein Verfahren zur Herstellung von N,N-Dimethyl-acetamid (DMAC) durch kontinuierliche Umsetzung von Methylacetat (MeOAc) mit Dimethylamin (DMA) in Gegenwart eines basischen Katalysators.

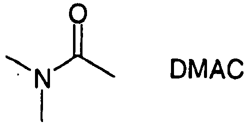

DMAC findet Verwendung als polares Lösungsmittel, z.B. für Polymere und für Gase, als Abbeizmittel, Extraktionsmittel, Katalysator und Kristallisationshilfsmittel. In der Lackindustrie wird DMAC wegen seiner hohen Siedetemperatur für spezielle Beschichtungsstoffe auf der Bindemittel-Basis von Polymeren, wie z.B. Polyamiden und Polyurethanen, eingesetzt. DMAC wird weiterhin zur Herstellung von Fasern und Folien und als Reaktionsmedium verwendet. DMAC wird beim Verspinnen von Spandex®-Fasern als Hilfsstoff eingesetzt und anschließend zumindest teilweise zurückgewonnen.

DMAC kann aus Essigsäure und Dimethylamin hergestellt werden, z.B. gemäß FR-A-1,406,279.

Carbonsäureamide sind auch durch Aminolyse von entsprechenden Carbonsäureestern zugänglich, vgl. z.B. ,Organikum', VEB Deutscher Verlag der Wissenschaften, 1963, Seiten 374-375.

Der Artikel von J.P. Guthrie in J. Am. Chem. Soc. 96, Seiten 3608-15 (1974), betrifft reaktionskinetische und thermodynamische Aspekte u.a. der Aminolyse von Carbonsäureestern.

CA-A-1 073 467 und CA-A-1 073 468 (beide General Electric Comp.) beschreiben die Herstellung von Diolen und N,N-Dialkylamiden durch Umsetzung von Carbonsäurediolestern mit Dialkylaminen.

US-A-4,258,200 (Air Products) lehrt die Herstellung von DMAC aus Methylacetat und DMA in Gegenwart von Cobalt-Katalysatoren.

Gemäß Beispiel 1 wird ein "20 % methanol-methylacetat azeotrope" zur Umsetzung bei 155 - 160°F (68,4 - 71,2°C) eingesetzt.

JP-A-02 160749 (Lion Akzo KK) betrifft gemäß den Patent Abstracts of Japan die Reaktion von aliphatischen Carbonsäureestern mit Ammoniak oder einem Amin, wie Monomethylamin, Ethylendiamin, Diethylentriamin, in Gegenwart eines "alkali catalyst" bei 50 bis 180°C, insbesondere 80 bis 160°C, und im Druckbereich von Normaldruck bis 0,981 MPa (9,81 bar) (10 kg • cm⁻² • G).

Es werden 0,1 bis 10 Mol.%, insbesondere 1 bis 5 Mol.%, bezogen auf den eingesetzten Carbonsäureester, Natriummethanolat (NaOMe) als Katalysator eingesetzt.

Derwent Abstract 84-016399/03 (SU-A-1 004 357; Dnepr Chem. Techn. Inst.) beschreibt die Herstellung von DMAC oder Dimethylformamid (DMF) durch Umsetzung eines 5-20 %igen Überschusses an entsprechendem Carbonsäuremethylester in Methanol mit DMA bei 50 -150°C und anschließendem Recycling von nicht abreagiertem Ester und Methanol in die Reaktionsstufe.

Im Beispiel wird eine Lösung von 0,4 kg Methylformiat in 0,2 kg Methanol / h kontinuierlich mit 0,2 kg dampfförmigen DMA / h zu DMF umgesetzt.

Die beiden deutschen Patentanmeldungen Nr. 102004030616.8 vom 24.06.04 und DE-A-10 315 214 von BASF AG betreffen Verfahren zur Reinigung von DMAC.

Die US 4,258,200 offenbart ein Verfahren zur Herstellung von Carbonsäureamiden durch Umsetzung von Carbonsäureestern mit primären oder sekundären Aminen in Gegenwart von Organo-Kobaltkatalysatoren.

Der vorliegenden Erfindung lag die Aufgabe zugrunde, ein verbessertes wirtschaftliches, selektives, energie- und ressourcensparendes Verfahren zur Herstellung von N,N-Dimethylacetamid (DMAC) aufzufinden. Das Verfahren sollte DMAC in hoher Ausbeute und Raum-Zeit-Ausbeute und in hoher Reinheit (z.B. frei oder quasi frei von Essigsäure, hohe Farbqualität) liefern.

Demgemäß wurde ein Verfahren zur Herstellung von N,N-Dimethylacetamid (DMAC) durch kontinuierliche Umsetzung von Methylacetat (MeOAc) mit Dimethylamin (DMA) in Gegenwart eines basischen Katalysators gefunden, welches dadurch gekennzeichnet, dass MeOAc als methanolische Lösung, die bei der Herstellung von PolyTHF durch Umesterung von PolyTHF-Diacetat mit Methanol als Nebenprodukt anfällt, eingesetzt wird.

Das erfindungsgemäße Verfahren lässt sich wie folgt ausführen:

Zur DMAC-Synthese wird Dimethylamin (DMA) mit einer methanolischen Lösung von Methylacetat (MeOAc), einem Nebensstrom der PolyTHF-Herstellung, kontinuierlich umgesetzt.

Pro Mol Methylacetat werden bevorzugt im Bereich von 0,2 bis 2,0 Mol, besonders 0,5 bis 1,5 Mol, ganz besonders 0,8 bis 1,2 Mol, z.B. 0,9 bis 1,1 Mol oder 1,0 bis 1,05 Mol, Dimethylamin (DMA) eingesetzt.

Das eingesetzte DMA weist bevorzugt eine Reinheit von ≥ 99 Gew.-%, insbesondere ≥ 99,4 Gew.-%, auf und liegt z.B. im Bereich von 99,5 bis 99,8 Gew.-%.

Die methanolische MeOAc-Lösung weist bevorzugt eine Konzentration im Bereich von 65 bis 90 Gew.-%, bevorzugt 70 bis 85 Gew.-%. insbesondere 75 bis 82 Gew.-%, MeOAc auf.

Als methanolische MeOAc-Lösung wird erfingdungsgemäß ein entsprechender Nebenproduktstrom, der bei der Produktion von PolyTHF (Polytetrahydrofuran), z.B. nach dem zweistufigen BASF-Verfahren gemäß EP-A-3112, DE-A-197 58 296 und/oder DE-A-198 17 113, anfällt, eingesetzt.

Die methanolische MeOAc-Lösung als entsprechender Nebenproduktstrom fällt bei der destillativen Aufarbeitung z.B. als Methylacetat/Methanol-Azeotrop (Siedepunkt: 54°C / 1013 hPa (1013 mbar)) an, da bei der Umesterung von PolyTHF-Diacetat (= Poly-(1,4-butandiol)-bis-(acetat)) mit Methanol zu PolyTHF stöchiometrische Mengen an MeOAc entstehen.

Bevorzugt weist die methanolische MeOAc-Lösung folgende Gehalte auf:

| | |
|---|---|
| MeOAc: | 65 bis 90 Gew.-%, bevorzugt 70 bis 85 Gew.-%, insbesondere 75 bis 82 Gew.-%, |
| Methanol: | 10 bis 30 Gew.-%, bevorzugt 14,8 bis 25 Gew.-%, insbesondere 17,6 bis 22 Gew.-%, |
| Dimethylether: | 0 bis 2 Gew.-%, bevorzugt 0,1 bis 1,5 Gew.-%, insbesondere 0,2 bis 1,2 Gew.-%, |
| THF: | 0 bis 4 Gew.-%, bevorzugt 0,1 bis 3,5 Gew.-%, insbesondere 0,2 bis 1,5 Gew.-%, und |
| H₂O: | 0 bis 0,1 Gew.-%, bevorzugt 0 bis 0,01 Gew.-%, insbesondere 0 bis 0,003 Gew.-%. |

Insbesondere besteht die methanolische MeOAc-Lösung aus MeOAc, MeOH, Dimethylether, THF und Wasser in den oben angegebenen Mengen.

Die kontinuierliche Umsetzung wird bevorzugt bei einem Absolutdruck im Bereich von 0,01 bis 20 MPa (1 bis 200 bar), bevorzugt 0,3 bis 10 MPa (3 bis 100 bar), insbesondere 1 bis 3 MPa (10 bis 30 bar), ganz besonders 1,2 bis 2,5 MPa (12 bis 25 bar), z.B. 1,5 bis 2,0 MPa (15 bis 20 bar), durchgeführt.

Die Reaktionstemperatur liegt bevorzugt im Bereich von 20 bis 200°C, bevorzugt 60 bis 140°C, insbesondere 80 bis 120°C, ganz besonders 90 bis 110°C, z.B. 95 bis 105°C.

Als Reaktoren für die erfindungsgemäße Umsetzung kommen insbesondere rückvermischende Reaktoren wie beispielsweise Rührkesselreaktoren oder Strahlschlaufenreaktoren, nicht-rückvermischende Reaktoren wie Rührkesselkaskaden oder Rohrreaktoren und Sonderbauformen wie Reaktionskolonnen mit und ohne innen oder außenliegenden VWZ-Volumina (VWZ = Verweilzeit) in Betracht, wobei eine innen und außenliegender Wärmeabfuhr möglich ist.

Die Umsetzung erfolgt besonders bevorzugt in einem Strahlschlaufenreaktor. Der Strahlschlaufenreaktor ist bevorzugt mit einem Einsteckrohr und unten liegender Düse ausgerüstet. Bevorzugt wird dabei DMA zusammen mit dem Katalysator durch den umgepumpten Treibstrahl und das MeOAc durch den Mantelstrahl zugegeben.

Zur Vervollständigung des Umsatzes wird dem Hauptreaktor, z.B. dem Strahlschlaufenreaktor, besonders bevorzugt ein Nachreaktor, z.B. ein Strömungsrohr oder ein kaskadierter Verweilzeitbehälter, nachgeschaltet.

Die genannten Reaktortypen sind dem Fachmann z.B. aus Ullmanns Enzyklopädie der Technischen Chemie, 4. Auflage, Band 13, S. 135 ff., und P.N. Rylander, "Hydrogenation and Dehydrogenation" in Ullmann's Encyclopedia of Industrial Chemistry, 5th ed. on CD-ROM bekannt.

Im erfindungsgemäßen Verfahren wird als basischer Katalysator bevorzugt ein Alkalimetallhydroxid, Erdalkalimetallhydroxid, Alkalimetallalkoholat, Erdalkalimetallalkoholat, Alkalimetallcarbonat, Erdalkalimetallcarbonat, Alkalimetallhydrogencarbonat, Erdatkalimetallhydrogencarbonat und/oder ein Amin, insbesondere tertiäres Amin, eingesetzt. Bei dem Alkalimetall handelt es sich um Li, Na, K, Rb oder Cs, insbesondere Na oder K.

Bei dem Erdalkalimetall handelt es sich um Be, Mg, Ca, Sr oder Ba, insbesondere Mg oder Ca.

Bei dem Alkoholat handelt es sich bevorzugt um ein C₁₋₄-Alkoholat, insbesondere Methanolat.

Bei dem insbesondere aliphatischen Amin handelt es sich bevorzugt um ein C₃₋₁₂-Alkylamin, wie z.B. Triethylamin, Tri-n-propylamin, Tri-n-butylamin, Dimethylethylamin, Diethylmethylamin, N-Methyl-piperidin, Triethylendiamin (TEDA).

Im erfindungsgemäßen Verfahren kommen keine Cobalt-Katalysatoren gemäß US-A-4,258,200 zum Einsatz.

Ganz besonders bevorzugter Katalysator im erfindungsgemäßen Verfahren ist Natriummethanolat (NaOMe).

Der Katalysator liegt im Reaktionsgemisch homogen und/oder suspendiert vor.

Bevorzugt werden im kontinuierlichen Verfahren pro Mol eingesetztem Methylacetat im Bereich von 0,0002 bis 0,09 Mol, bevorzugt 0,002 bis 0,05 Mol, insbesondere 0,003 bis 0,02 Mol, des Katalysators bzw. der Katalysatormischung eingesetzt.

Der Katalysator oder die Katalysatormischung wird vorteilhaft als Lösung und/oder Suspension in einem Lösungs- oder Suspensionsmittel eingesetzt.

Bevorzugte Lösungs- und/oder Suspensionsmittel sind Wasser und Alkohole (z.B. C₁₋₄-Alkohole, wie Methanol, Ethanol, n-Propanol, n-Butanol) oder Mischungen hiervon.

Bevorzugt ist im Falle eines Alkalimetallalkoholats als Katalysator das Alkalimetallalkoholat in dem Alkohol, der dem Alkoholat durch Protonierung entspricht, gelöst.

Der Katalysator oder die Katalysatormischung wird in den o.g. bevorzugten Mengen bevorzugt als 1 bis 35 Gew.-%ige, insbesondere 5 bis 30 Gew.-%ige, Lösung oder Suspension eingesetzt.

Besonders vorteilhaft wird als Katalysator NaOMe in den o.g. bevorzugten Mengen als methanolische Lösung, insbesondere als 1 bis 35 Gew.-%ige Lösung, ganz besonders als 25 bis 30 Gew.-%ige Lösung, eingesetzt.

Die Umsetzung des MeOAc im erfindungsgemäßen Verfahren wird bevorzugt in Gegenwart von weniger als 1 Gew.-%, besonders weniger als 0,5 Gew.-%, ganz besonders im Bereich von 0 bis 0,3 Gew.-%, Wasser, jeweils bezogen auf das Gewicht der beiden Einsatzstoffe MeOAc und DMA (in Summe), durchgeführt.

Die Abfuhr der Reaktionswärme der Umsetzung erfolgt bevorzugt über einen externen Wärmetauscher. Besonders vorteilhaft wird der im externen Wärmetauscher erzeugte Dampf, z.B. 0,15 MPa (1,5-bar)-Dampf, in einer Syntheseanlage für Methylamine aus Methanol und Ammoniak genutzt.

Im erfindungsgemäßen Verfahren besteht der flüssige Reaktoraustrag aus der Synthesestufe aus

im Bereich von 45 bis 74,5 Gew.-%, besonders 50 bis 70 Gew.-%, DMAC,

im Bereich von 25 bis 45 Gew.-%, besonders 29 bis 40 Gew.-%, Methanol und insgesamt 0,5 bis 6 Gew.-%, besonders 1 bis 5 Gew.-%, DMA, Methylacetat, Katalysator (z.B. Natriummethanolat), ggf. Katalysatorlösungsmittel/-suspensionsmittel und Nebenprodukte.

Durch den Einsatz von methanolischer MeOAc-Lösung, die bei der Produktion von Poly-THF anfällt, können Tetrahydrofuran (THF) und/oder Dimethylether solche Nebenprodukte sein.

Zur weiteren Aufarbeitung kann der flüssige Reaktoraustrag direkt in eine Blase einer Destillationskolonne entspannt werden. In einer besonderen Ausführungsform wird in zwei alternierend betriebene Destillationsblasen entspannt.

Dem Austrag wird vorteilhaft Wasser oder eine wässrige oder wasserfreie Protonensäure, wie Schwefelsäure, Methansulfonsäure, Carbonsäure (z.B. C₁₋₄-Carbonsäure), insbesondere Phosphorsäure, bevorzugt in einer Menge die einen vollständigen Umsatz des eingesetzten basischen Katalysators zur korrespondierenden Säure und zum entsprechenden Alkalimetall-, Erdalkalimetall- oder Ammonium-Salz der Protonensäure gewährleistet, zugesetzt. D.h. bevorzugt wird der eingesetzte und im Reaktoraustrag vorhandene basische Katalysator durch Umsetzung mit H⁺ vollständig neutralisiert.

Dies ist vorteilhaft, da der basische Katalysator, wie z.B. Natriummethanolat, nach der Ausgasung von restlichem DMA die Rückspaltung von DMAC katalysieren würde.

Das organische Produktgemisch wird bevorzugt durch Verdampfen (bei Normaldruck oder im Vakuum, z.B. in einem Aufkocher) von vorhandenen Salzen abgetrennt, z.B. bis ein ausfallendes Salz die Wärmetauscherleistung deutlich reduziert und zu Verkrustungen führt.

Die Blase für den Reaktoraustrag wird dann bevorzugt gewechselt und der Rückstand der alten Blase möglichst weit eingedampft. Der ausgefallene feste Salzrückstand kann in Wasser aufgelöst und als Lösung in eine Kläranlage entsorgt werden.

Der vom Feststoff abgedampfte und partiell- oder total-kondensierte Reaktoraustrag wird destillativ aufgearbeitet, z.B. in einer, zwei, drei, vier oder mehr, ggf. miteinander verschaltenen, Kolonnen.

Bevorzugt wird in drei kontinuierlich betriebenen Destillationskolonnen aufgearbeitet.

Dabei werden zunächst in einer Kolonne A bei bevorzugt 0,08 bis 0,12 MPa (0,8 bis 1,2 bar) Methanol und ggf. andere Leichtsieder (DMA, Wasser, THF, Methylacetat u. a.) über Kopf abgetrennt.

In der bevorzugt nachgeschalteten Destillationskolonne D zur Leichtsiederaufreinigung wird ein ggf. wässriger Methanolstrom, der DMA enthalten kann, angereichert, der z.B. vorteilhaft zum Einsatz in eine Methylamine-Syntheseanlage (insbesondere zur DMA-Herstellung) rückgeführt wird.

Der Sumpfaustrag der Kolonne A wird einer Kolonne B zugeführt. Bei bevorzugt 100-500 hPa (mbar) abs. wird hier reines DMAC (≥ 99,5 Gew.-%, insbesondere ≥ 99,7 Gew.-%, ganz besonders ≥ 99,8 Gew.-%, z.B. im Bereich von ≥ 99,9 bis 99,99 Gew.-%), bevorzugt über einen flüssigen Seitenabzug, der sich bevorzugt im Verstärkungsteil befindet, abgetrennt.

Der Kopfaustrag der Kolonne B, enthaltend DMAC (z.B. ≥98 Gew.-% DMAC, insbesondere 98,5 bis 99,5 Gew.-% DMAC), wird bevorzugt in die Kolonne A zurückgeführt.

Der Sumpfaustrag der Kolonne B wird in einer Kolonne C bei bevorzugt Normaldruck nochmals aufgetrennt, wobei der Kopfaustrag, enthaltend DMAC und Methanol (z.B. ca. 94 Gew.-% DMAC und ca. 6 Gew.-% Methanol) bevorzugt ebenfalls zur Kolonne A zurückgeführt wird und der Sumpfaustrag der Kolonne C (Hochsieder, DMAC und zugesetztes Methanol) zur Entsorgung, z.B. Verbrennung, gelangt. Durch die dritte Kolonne C wird die Rückstandsmenge deutlich reduziert.

Die destillative Reinigung von DMAC kann auch gemäß einem der Verfahren der beiden deutschen Patentanmeldungen Nr. 102004030616.8 vom 24.06.04 und DE-A-10 315 214 (beide BASF AG) erfolgen.

Erfindungsgemäß wurde erkannt, dass das Verfahren vorteilhaft auch in einer Anlage durchgeführt werden kann, die ursprünglich für die Herstellung von N,N-Dimethylformamid (DMF) aus Kohlenmonoxid (CO) und DMA konzipiert ist.

Durch geringfügige Modifikationen/Anlageergänzungen (wie z.B. Nachreaktor, Tank für DMAC und/oder betreffend die Kolonnenverschaltung) kann so in der DMF-Anlage, wie sie z.B. in K. Weissermel, H.-J. Arpe, Industrielle Organische Chemie, Wiley-VCH, 5. Auflage 1998, Seite 49, oder allgemein und prinzipiell in JP-A2-110 92 434 beschrieben ist, vorteilhaft sowohl DMF als auch DMAC, z.B. im Wechselbetrieb, hergestellt werden. D.h. die Erfindung ermöglicht auch die alternative oder alternierende Produktion von DMAC in einer DMF-Anlage.

Mit dem erfindungsgemäßen Verfahren werden DMAC-Ausbeuten im Bereich von ≥ 88 %, insbesondere ≥ 95 %, ganz besonders ≥ 99 %, z.B. 99,5 bis 99,9 %, (jeweils bezogen auf eingesetztes MeOAc), bei MeOAc-Umsätzen im Bereich von ≥ 90 %, insbesondere ≥ 96 %, ganz besonders ≥ 99 %, z.B. 99,5 bis 100 %, erzielt.

Die DMAC-Raum-Zeit-Ausbeuten liegen im Bereich von 0,1 bis 0,85 kg DMAC / (Liter Reaktorvolumen • h), z.B. 0,2 bis 0,5 kg DMAC / (Liter Reaktorvolumen • h).

Das erfindungsgemäße Verfahren liefert DMAC mit einer Reinheit von ≥ 99,5 Gew.-%, insbesondere ≥ 99,7 Gew.-%, ganz besonders ≥ 99,8 Gew.-%, z.B. im Bereich von ≥ 99,9 bis 99,99 Gew.-%, (Methode und Bedingungen zur Reinheitsbestimmung s.u.),
einem Wassergehalt ≤ 200 ppm, z.B. im Bereich von 50 bis 150 ppm, (nach DIN 51777), und
einer Pt/Co-Farbzahl ≤ 10, besonders ≤ 8, z. B. im Bereich von 1 bis 6, (nach DIN ISO 6271).

Der Säuregehalt (berechnet als Essigsäure) des DMAC liegt insbesondere bei ≤ 80 ppm, ganz besonders bei ≤ 70 ppm, z.B. im Bereich von 5 bis 60 ppm, (nach DIN 53402).

Alle ppm-Angaben in diesem Dokument beziehen sich auf das Gewicht (Gew.-ppm).

### Beispiele

### Beispiel 1

Zur einstufigen DMAC-Synthese wurden 45,0 g/h Dimethylamin (DMA) mit 95,5 g/h methanolischem Methylacetat (77,5 Gew.-%ig), welches zuvor als Nebenproduktstrom bei der Produktion von Poly-THF gemäß EP-A-3112, DE-A-197 58 296 und/oder DE-A-198 17 113 anfiel (THF-Gehalt: 1,5 Gew.-%), bei 2,0 MPa (20 bar) und 120°C umgesetzt. Der Wassergehalt im Zulauf (DMA + methanolisches Methylacetat) betrug 109 ppm.

Die Reaktion erfolgte in einem Schlaufenreaktor mit einer mittleren Verweilzeit (VWZ) von 1 h und Natriummethanolat (0,48 g/h) in methanolischer Lösung (30 Gew.-%ig) als homogenem Katalysator. Die Wärmeabfuhr erfolgte über einen externen Wärmetauscher. Die im externen Wärmetauscher abgeführte Energie kann 0,15 MPA (1,5-bar)-Dampf erzeugen.

Der flüssige Austrag aus der Synthesestufe bestand aus 57,7 Gew.-% DMAC, 34,2 Gew.-% Methanol, 5,0 Gew.-% Methylacetat und insgesamt 3,1 Gew.-% DMA, Tetrahydrofuran, Natriummethanolat und Nebenprodukte.

### Beispiel 2

Es wurden alle Einstellungen aus Beispiel 1 übernommen. Allerdings betrug der Wassergehalt des Zulaufstroms 550 ppm. Nach kurzer Zeit kam es im Reaktor durch ausfallendes Natriumacetat zu Verstopfungen und der Versuch musste abgebrochen werden.

### Beispiel 3

Zur zweistufigen DMAC-Synthese wurden 45,2 g/h Dimethylamin (DMA) mit 92,5 g/h methanolischem Methylacetat (78,8 Gew.-%ig), welches zuvor als Nebenproduktstrom bei der Produktion von Poly-THF gemäß EP-A-3112, DE-A-197 58 296 und/oder DE-A-198 17 113 anfiel (THF-Gehalt: 1,0 Gew.-%), bei 2 MPa (20 bar) und 120°C umgesetzt.

Die Reaktion erfolgte in einem Schlaufenreaktor mit einer mittleren VWZ von 1 h und Natriummethanolat (0,56 g/h) in methanolischer Lösung (30 Gew.-%ig) als homogenem Katalysator. Die Wärmeabfuhr erfolgte über einen externen Wärmetauscher. Die im externen Wärmetauscher abgeführte Energie kann 0,15 MPa (1,5-bar)-Dampf erzeugen.

Der flüssige Austrag aus der Synthesestufe bestand aus 53,9 Gew.-% DMAC, 36,3 Gew.-% Methanol, 3,9 Gew.-% Methylacetat und insgesamt 5,9 Gew.-% DMA, Tetrahydrofuran, Natriummethanolat und Nebenprodukte.

Dieser Austrag wurde im geraden Durchgang durch einen Rohrreaktor bei 120°C, 2 MPa (20 bar) und einer mittleren VWZ von 1 h gefördert. Der Austrag bestand aus 58,3 Gew.-% DMAC, 37,3 Gew.-% Methanol, 1,1 Gew.-% Methylacetat und insgesamt 3,3 Gew.-% DMA, Tetrahydrofuran, Natriummethanolat und Nebenprodukte.

### Beispiel 4

Einem Reaktionsaustrag nach Beispiel 3 wurde kontinuierlich 10 Gew.-% überstöchiometrisch zum Katalysator H₂O zugesetzt, um das Natriummethanolat zu zersetzen. In einer kontinuierlichen Abdampfblase wurden bei 135°C alle flüchtigen Inhaltsstoffe (1,8 kg/h) abdestilliert. Der sich innerhalb von 20 Betriebsstunden angesammelte und bis zur Trockene eingedampfte salzartige Rückstand (245 g) im Sumpf der Blase wurde in 1,5 kg H₂O gelöst und rückstandsfrei aus der Blase ins behandlungsbedürftige Abwasser (bbA) abgeführt.

### Beispiel 5

Ein Reaktionsaustrag nach Beispiel 3 wurde kontinuierlich mit 85 %iger Phosphorsäure zur stöchiometrischen Bildung von Na2HPO4 versetzt. Nach erfolgter Katalysatorzersetzung und Abdampfung der flüchtigen Inhaltsstoffe gemäß Beispiel 4 wurden 400 g/h des kondensierten Gemisches kontinuierlich einer Destillationskolonne zugeführt und bei einer Sumpftemperatur von 175°C ein Schwersiederstrom (218 g/h) mit 99,2 Gew.-% DMAC und 0,8 Gew.-% Nebenprodukte abgezogen. In einer nachfolgen den kontinuierlichen Destillation wurde dieser Strom weiter aufgearbeitet, wobei aus einem Seitenabzug 198 g/h DMAC mit einer Reinheit von 99,9 % erhalten wurde.

## Patentansprüche

1. Verfahren zur Herstellung von N,N-Dimethylacetamid (DMAC) durch kontinuierliche Umsetzung von Methylacetat (MeOAc) mit Dimethylamin (DMA) in Gegenwart eines basischen Katalysators, **dadurch gekennzeichnet, dass** MeOAc als methanolische Lösung, die bei der Herstellung von PolyTHF durch Umesterung von PolyTHF-Diacetat mit Methanol als Nebenprodukt anfällt, eingesetzt wird.

2. Verfahren nach Anspruch 1, **dadurch gekennzeichnet, dass** man die Umsetzung bei einer Temperatur im Bereich von 80 bis 140°C durchführt.

3. Verfahren nach den Ansprüchen 1 oder 2, **dadurch gekennzeichnet, dass** man die Umsetzung bei einem Absolutdruck im Bereich von 3 bis 30 bar durchführt.

4. Verfahren nach einem der vorhergehenden Ansprüche, **dadurch gekennzeichnet, dass** die methanolische MeOAc-Lösung 70 bis 85 Gew.-% MeOAc, 14,8 bis 25 Gew.-% Methanol, 0,1 bis 1,5 Gew.-% Dimethylether, 0,1 bis 3,5 Gew.-% Tetrahydrofuran (THF) und 0 bis 0,01 Gew.-% Wasser enthält.

5. Verfahren nach einem der Ansprüche 1 bis 3, **dadurch gekennzeichnet, dass** die methanolische MeOAc-Lösung 75 bis 82 Gew.-% MeOAc, 17,6 bis 22 Gew.-% Methanol, 0,2 bis 1,2 Gew.-% Dimethylether, 0,2 bis 1,5 Gew.-% Tetrahydrofuran (THF) und 0 bis 0,003 Gew.-% Wasser enthält.

6. Verfahren nach einem der vorhergehenden Ansprüche, **dadurch gekennzeichnet, dass** als Katalysator Natriummethanolat eingesetzt wird.

7. Verfahren nach dem vorhergehenden Anspruch, **dadurch gekennzeichnet, dass** der Katalysator als methanolische Lösung eingesetzt wird.

8. Verfahren nach einem der vorhergehenden Ansprüche, **dadurch gekennzeichnet, dass** pro Mol Methylacetat im Bereich von 0,0002 bis 0,09 Mol Katalysator eingesetzt wird.

9. Verfahren nach einem der vorhergehenden Ansprüche, **dadurch gekennzeichnet, dass** die Umsetzung in einem Strahlschlaufenreaktor durchgeführt wird.

10. Verfahren nach dem vorhergehenden Anspruch, **dadurch gekennzeichnet, dass** der Strahlschlaufenreaktor ein Einsteckrohr und eine unten liegende Düse aufweist.

11. Verfahren nach einem der vorhergehenden Ansprüche, **dadurch gekennzeichnet, dass** nach der Umsetzung und vor der destillativen Aufarbeitung der im Reaktoraustrag vorhandene basische Katalysator durch Umsetzung mit einer Protonensäure neutralisiert oder mit Wasser zersetzt wird.

12. Verfahren nach dem vorhergehenden Anspruch, **dadurch gekennzeichnet, dass** es sich bei der Protonensäure um Phosphorsäure handelt.

13. Verfahren nach einem der beiden vorhergehenden Ansprüche, **dadurch gekennzeichnet, dass** nach der Umsetzung, nach der Neutralisation mit einer Protonensäure und vor der destillativen Aufarbeitung das organische Produktgemisch durch Verdampfen von vorhandenen Salzen abgetrennt wird.

14. Verfahren nach einem der vorhergehenden Ansprüche, **dadurch gekennzeichnet, dass** die kontinuierliche destillative Aufarbeitung derart erfolgt, dass zunächst in einer Kolonne A Methanol und gegebenenfalls andere Leichtsieder über Kopf abgetrennt werden und der Sumpfaustrag der Kolonne A einer Kolonne B zugeführt wird, in der DMAC mit einer Reinheit von ≥ 99,7 Gew.-% über einen Seitenabzug abgetrennt wird.

15. Verfahren nach dem vorhergehenden Anspruch, **dadurch gekennzeichnet, dass** in der Kolonne B das DMAC mit einer Reinheit von ≥ 99,7 Gew.-% über einen flüssigen Seitenabzug, der sich im Verstärkungsteil der Kolonne befindet, abgetrennt wird.

16. Verfahren nach einem der beiden vorhergehenden Ansprüche, **dadurch gekennzeichnet, dass** der Kopfaustrag der Kolonne B, enthaltend DMAC, in die Kolonne A zurückgeführt wird.

17. Verfahren nach einem der drei vorhergehenden Ansprüche, **dadurch gekennzeichnet, dass** der Sumpfaustrag der Kolonne B in einer Kolonne C aufgetrennt wird, wobei der Kopfaustrag, enthaltend DMAC und Methanol, zur Kolonne A zurückgeführt wird.

18. Verfahren nach einem der vier vorhergehenden Ansprüche, **dadurch gekennzeichnet, dass** der Kopfaustrag der Kolonne A, enthaltend Methanol, in einer Kolonne D aufgereinigt und in einer Syntheseanlage zur Herstellung von Methylaminen aus Methanol und Ammoniak eingesetzt wird.

19. Verfahren nach einem der vorhergehenden Ansprüche zur Herstellung von DMAC mit einer Reinheit von ≥ 99,7 Gew.-%, einem Wassergehalt ≤ 200 ppm und einer Pt/Co-Farbzahl ≤ 10.

20. Verfahren nach einem der vorhergehenden Ansprüche zur Herstellung von DMAC mit einem Säuregehalt (berechnet als Essigsäure) von ≤ 80 ppm.

21. Verfahren nach einem der vorhergehenden Ansprüche, **dadurch gekennzeichnet, dass** es in einer Anlage durchgeführt wird, in der auch N,N-Dimethylformamid (DMF) aus Kohlenmonoxid (CO) und DMA hergestellt werden kann.

## Claims

1. A process for preparing N,N-dimethylacetamide (DMAC) by continuously reacting methyl acetate (MeOAc) with dimethylamine (DMA) in the presence of a basic catalyst, which comprises using MeOAc in the form of a methanolic solution which is obtained as a by-product in the preparation of polyTHF by transesterifying polyTHF diacetate with methanol.

2. The process according to claim 1, wherein the reaction is carried out at a temperature in the range from 80 to 140°C.

3. The process according to claim 1 or 2, wherein the reaction is carried out at an absolute pressure in the range from 3 to 30 bar.

4. The process according to any of the preceding claims, wherein the methanolic MeOAc solution comprises from 70 to 85% by weight of MeOAc, from 14.8 to 25% by weight of methanol, from 0.1 to 1.5% by weight of dimethyl ether, from 0.1 to 3.5% by weight of tetrahydrofuran (THF) and from 0 to 0.01% by weight of water.

5. The process according to any of claims 1 to 3, wherein the methanolic MeOAc solution comprises from 75 to 82% by weight of MeOAc, from 17.6 to 22% by weight of methanol, from 0.2 to 1.2% by weight of dimethyl ether, from 0.2 to 1.5% by weight of tetrahydrofuran (THF) and from 0 to 0.003% by weight of water.

6. The process according to any of the preceding claims, wherein the catalyst used is sodium methoxide.

7. The process according to the preceding claim, wherein the catalyst is used in the form of a methanolic solution.

8. The process according to any of the preceding claims, wherein catalyst is used in the range from 0.0002 to 0.09 mol per mole of methyl acetate.

9. The process according to any of the preceding claims, wherein the reaction is carried out in a jet loop reactor.

10. The process according to the preceding claim, wherein the jet loop reactor has an insert tube and a nozzle at the bottom.

11. The process according to any of the preceding claims, wherein the basic catalyst present in the reactor effluent is neutralized by reacting with a protic acid or decomposed with water after the reaction and before the distillative workup.

12. The process according to the preceding claim, wherein the protic acid is phosphoric acid.

13. The process according to either of the two preceding claims, wherein the organic product mixture is removed by evaporation from salts present after the reaction, after the neutralization with a protic acid and before the distillative workup.

14. The process according to any of the preceding claims, wherein the continuous distillative workup is effected in such a way that methanol and any other low boilers are initially removed overhead in a column A and the bottom effluent of column A is fed to a column B in which DMAC is removed via a side draw with a purity of ≥ 99.7% by weight.

15. The process according to the preceding claim, wherein the DMAC is removed in column B with a purity of ≥ 99.7% by weight via a liquid side draw which is disposed in the rectifying section of the column.

16. The process according to either of the two preceding claims, wherein the top effluent of column B, comprising DMAC, is recycled into column A.

17. The process according to any of the three preceding claims, wherein the bottom effluent of column B is separated in a column C, and the top effluent comprising DMAC and methanol is recycled to column A.

18. The process according to one of the four preceding claims, wherein the top effluent of column A, comprising methanol, is purified in a column D and is used in a synthesis plant for preparing methylamines from methanol and ammonia.

19. The process according to any of the preceding claims for preparing DMAC having a purity of ≥ 99.7% by weight, a water content ≤ 200 ppm and a Pt/Co color number ≤ 10.

20. The process according to any of the preceding claims for preparing DMAC having an acid content (calculated as acetic acid) of ≤ 80 ppm.

21. The process according to any of the preceding claims, which is carried out in a plant in which N,N-dimethylformamide (DMF) can also be prepared from carbon monoxide (CO) and DMA.

## Revendications

1. Procédé pour la production de N,N-diméthylacétamide (DMAC) par mise en réaction continue d'acétate de méthyle (MeOAc) avec de la diméthylamine (DMA) en présence d'un catalyseur basique, **caractérisé en ce qu'**on utilise le MeOAc sous forme d'une solution méthanolique qui apparaît en tant que sous-produit dans la production de polyTHF par transestérification de diacétate de polyTHF avec du méthanol.

2. Procédé selon la revendication 1, **caractérisé en ce qu'**on effectue la réaction à une température dans la plage de 80 à 140 °C.

3. Procédé selon la revendication 1 ou 2, **caractérisé en ce qu'**on effectue la réaction sous une pression absolue dans la plage de 3 à 30 bars.

4. Procédé selon l'une quelconque des revendications précédentes, **caractérisé en ce que** la solution méthanolique de MeOAc contient 70 à 85 % en poids de MeOAc, 14,8 à 25 % en poids de méthanol, 0,1 à 1,5 % en poids d'éther diméthylique, 0,1 à 3,5 % en poids de tétrahydrofurane (THF) et 0 à 0,01 % en poids d'eau.

5. Procédé selon l'une quelconque des revendications 1 à 3, **caractérisé en ce que** la solution méthanolique de MeOAc contient 75 à 82 % en poids de MeOAc, 17,6 à 22 % en poids de méthanol, 0,2 à 1,2 % en poids d'éther diméthylique, 0,2 à 1,5 % en poids de tétrahydrofurane (THF) et 0 à 0,003 % en poids d'eau.

6. Procédé selon l'une quelconque des revendications précédentes, **caractérisé en ce qu'**on utilise comme catalyseur le méthanolate de sodium.

7. Procédé selon la revendication précédente, **caractérisé en ce qu'**on utilise le catalyseur sous forme de solution méthanolique.

8. Procédé selon l'une quelconque des revendications précédentes, **caractérisé en ce qu'**on utilise dans la plage de 0,0002 à 0,09 mole de catalyseur par mole d'acétate de méthyle.

9. Procédé selon l'une quelconque des revendications précédentes, **caractérisé en ce qu'**on effectue la réaction dans un réacteur à jet à circulation en boucle.

10. Procédé selon la revendication précédente, **caractérisé en ce que** le réacteur à jet à circulation en boucle comprend un tube inséré et une buse se trouvant au-dessous.

11. Procédé selon l'une quelconque des revendications précédentes, **caractérisé en ce qu'**après la réaction et avant le traitement final par distillation on neutralise par mise en réaction avec un acide protonique le catalyseur basique présent dans le courant de décharge du réacteur ou on y ajoute de l'eau.

12. Procédé selon la revendication précédente, **caractérisé en ce que** l'acide protonique consiste en acide phosphorique.

13. Procédé selon l'une quelconque des deux revendications précédentes, **caractérisé en ce qu'**après la réaction, après la neutralisation avec un acide protonique et avant le traitement final par distillation on sépare par évaporation le mélange de produits organiques d'avec les sels présents.

14. Procédé selon l'une quelconque des revendications précédentes, **caractérisé en ce qu'**on effectue le traitement final par distillation en continu d'abord en séparant par la tête dans une colonne A le méthanol et éventuellement d'autres composants à bas point d'ébullition et en envoyant le courant de décharge de pied de la colonne A à une colonne B, dans laquelle le DMAC est séparé à un degré de pureté de ≥ 99,7 % en poids par un conduit latéral de décharge.

15. Procédé selon la revendication précédente, **caractérisé en ce que** dans la colonne B le DMAC est séparé à un degré de pureté de ≥ 99,7 % en poids par un conduit latéral de décharge liquide qui se trouve dans la partie de renforcement de la colonne.

16. Procédé selon l'une quelconque des deux revendications précédentes, **caractérisé en ce que** le courant de décharge de tête de la colonne B, contenant du DMAC, est renvoyé dans la colonne A.

17. Procédé selon l'une quelconque des trois revendications précédentes, **caractérisé en ce que** le courant de décharge de pied de la colonne B est fractionné dans une colonne C, dans laquelle le courant de décharge de tête, contenant du DMAC et du méthanol, est renvoyé à la colonne A.

18. Procédé selon l'une quelconque des quatre revendications précédentes, **caractérisé en ce que** le courant de décharge de tête de la colonne A, contenant du méthanol, est purifié dans une colonne D et utilisé dans une unité de synthèse pour la production de méthylamines à partir de méthanol et ammoniac.

19. Procédé selon l'une quelconque des revendications précédentes, pour la production de DMAC ayant un degré de pureté de ≥ 99,7 % en poids, une teneur en eau ≤ 200 ppm et un indice colorimétrique Pt/Co ≤ 10.

20. Procédé selon l'une quelconque des revendications précédentes, pour la production de DMAC ayant une teneur en acide (calculée en tant qu'acide acétique) de ≤ 80 ppm.

21. Procédé selon l'une quelconque des revendications précédentes, **caractérisé en ce qu'**il est mis en oeuvre dans une unité dans laquelle du N,N-diméthylformamide (DMF) peut également être produit à partir de monoxyde de carbone (CO) et DMA.
